(19) Europäisches Patentamt
European Patent Office
Office européen des brevets

(11) Publication number: **0 463 989 B1**

(12) # EUROPEAN PATENT SPECIFICATION

(45) Date of publication of patent specification: **28.12.94**

(51) Int. Cl.5: **C07C 259/10**, C07C 259/06, A61K 31/15

(21) Application number: **91810448.0**

(22) Date of filing: **12.06.91**

(54) **N-oxyimidic acid derivatives.**

(30) Priority: **13.06.90 US 537407**

(43) Date of publication of application:
**02.01.92 Bulletin 92/01**

(45) Publication of the grant of the patent:
**28.12.94 Bulletin 94/52**

(84) Designated Contracting States:
**AT BE CH DE DK ES FR GB GR IT LI LU NL SE**

(56) References cited:
**GB-A- 1 540 028**
**US-A- 3 649 664**

(73) Proprietor: **SANDOZ LTD.**
**Lichtstrasse 35**
**CH-4002 Basel (CH)**
(84) Designated Contracting States:
**BE CH DK ES FR GB GR IT LI LU NL SE**

(73) Proprietor: **SANDOZ-PATENT-GMBH**
**Humboldtstrasse 3**
**D-79539 Lörrach (DE)**
(84) Designated Contracting States:
**DE**

(73) Proprietor: **SANDOZ-ERFINDUNGEN Verwaltungsgesellschaft m.b.H.**
**Brunner Strasse 59**
**A-1230 Wien (AT)**
(84) Designated Contracting States:
**AT**

(72) Inventor: **Kapa, Prasad Koteswara**
**4 Faber Road**
**Parsippany, N.J. 07054 (US)**
Inventor: **Lee, George Tien-San**
**16 Macleod Lane**
**Bloomfiled, N.J. 07003 (US)**
Inventor: **Nadelson, Jeffrey**
**12 Benedict Crescent**
**Denville, N.J. 07834 (US)**
Inventor: **Simpson, William Ronald James**
**4 Linden Lane**
**Mendham, N.J. 07945 (US)**
Inventor: **Sunay, Ustan Bekir**
**46 Railroad Avenue**
**Netcong, N.J. 07857 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to substituted N-oxyimidic acid derivatives.

It concerns a compound of formula I

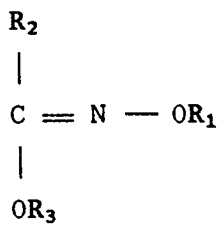

I

wherein

$R_1$ is: alkyl of 1 to 6 carbon atoms; alkenyl of 3 to 5 carbon atoms wherein the double bond is separated from the oxygen atom by at least 2 carbon atoms; mono- or polyhydroxyalkyl of 2 to 8 carbon atoms having up to 7 hydroxy groups, wherein the hydroxy groups are separated by at least 2 carbon atoms from the oxygen atom to which $R_1$ is bound; mono- or polyalkoxyalkyl of 1 to 4 carbon atoms in the alkoxy groups and of 2 to 8 carbon atoms in the alkylene group thereof having up to 7 alkoxy groups, wherein the alkoxy groups are separated by at least 2 carbon atoms from the oxygen atom to which $R_1$ is bound; phenyl or straight-chained phenylalkyl of 7 to 11 carbon atoms, both optionally mono- or independently disubstituted in the phenyl ring by halogen of atomic number of from 9 to 53, hydroxy, alkyl of 1 to 4 carbon atoms or alkoxy of 1 to 4 carbon atoms; carboxyalkyl of altogether 2 to 6 carbon atoms; alkoxycarbonylalkyl of 1 to 4 carbon atoms in the alkoxy group and of altogether 2 to 6 carbon atoms in the carbonylalkyl group thereof; or 4-pivaloylbenzyl;

either:

$R_2$ is: alkyl of 1 to 4 carbon atoms; phenyl or straight-chained phenylalkyl of 7 to 11 carbon atoms, both either optionally mono-or independently disubstituted in the phenyl ring by halogen of atomic number of from 9 to 53, hydroxy, alkyl of 1 to 4 carbon atoms or alkoxy of 1 to 4 carbon atoms, or monosubstituted in the 4 position of the phenyl ring by pivaloyl; and

$R_3$ is: 4-pivaloylbenzoyl;

or:

$R_2$ is: 4-pivaloylphenyl and

$R_3$ is: alkyl of 1 to 4 carbon atoms; straight-chained phenylalkyl of 7 to 11 carbon atoms, either optionally mono- or independently disubstituted in the phenyl ring by halogen of atomic number of from 9 to 53, hydroxy, alkyl of 1 to 4 carbon atoms or alkoxy of 1 to 4 carbon atoms, or monosubstituted in the 4 position of the phenyl ring by pivaloyl; alkylcarbonyl of altogether 2 to 5 carbon atoms; or benzoyl optionally mono- or independently disubstituted by halogen of atomic number of from 9 to 53, hydroxy, alkyl of 1 to 4 carbon atoms or alkoxy of 1 to 4 carbon atoms;

in free form or salt or pharmaceutically acceptable and physiologically hydrolysable ester form as appropriate.

$R_1$ preferably is alkyl as defined above, alkenyl as defined above, hydroxyalkyl as defined above, alkoxyalkyl as defined above, phenyl optionally substituted as defined above, or 4-pivaloylbenzyl, especially alkyl.

$R_2$ preferably is alkyl as defined above or phenyl optionally substituted as defined above, especially phenyl substituted as defined above, particularly 4-pivaloylphenyl.

$R_3$ preferably is alkyl as defined above, unsubstituted phenylalkyl as defined above, alkylcarbonyl as defined above or optionally substituted benzoyl as defined above, especially optionally substituted benzoyl, particularly 4-pivaloylbenzoyl.

Alkyl of 1 to 6 carbon atoms preferably is of 1 to 4 carbon atoms. Alkyl of 1 to 4 carbon atoms preferably is methyl, ethyl or tert-butyl, it especially is methyl. Alkenyl preferably is allyl. Hydroxyalkyl preferably is mono-, di- or trihydroxyalkyl, preferably mono- or dihydroxyalkyl, it especially is 2-hydroxyethyl or 2,3-dihydroxypropyl. It preferably has 2 to 6 carbon atoms. Alkoxyalkyl preferably is mono-, di-or trialkoxyalkyl, preferably mono- or dialkoxyalkyl, it especially is 2-methoxyethyl. It preferably has 2 to 6

carbon atoms in the alkylene group. Phenylalkyl preferably is benzyl. A phenyl ring preferably is unsubstituted or monosubstituted. A phenyl ring substituent preferably is in the 3 or 4 position, especially in the 4 position. A preferred phenyl ring substituent is pivaloyl; Halogen preferably is chlorine or bromine, especially chlorine. Alkoxy preferably is methoxy. Alkylcarbonyl preferably is acetyl or pivaloyl. Optionally substituted benzoyl preferably either is unsubstituted or is monosubstituted, preferably in the 4 position. Carboxyalkyl preferably is of altogether 2 or 3 carbon atoms, it especially is carboxymethyl. The alkoxy part of alkoxycarbonylalkyl preferably is methyl or ethyl; the carbonylalkyl part of alkoxycarbonylalkyl preferably is of altogether 2 or 3 carbon atoms, it especially is carbonylmethyl.

A pharmaceutically acceptable and physiologically hydrolysable ester form is e.g. the benzyl or a $C_{1-4}$alkyl ester, preferably the methyl ester.

A salt preferably is a pharmaceutically acceptable salt such as the sodium salt.

A subgroup of compounds of formula I is the compounds of formula Is

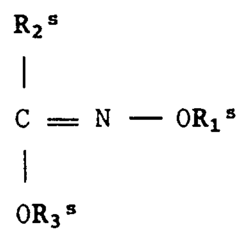

Is

wherein

$R_1{}^s$ is: alkyl of 1 to 4 carbon atoms; allyl; mono- or dihydroxyalkyl of 2 to 4 carbon atoms wherein the hydroxy groups are separated by at least 2 carbon atoms from the oxygen atom to which $R_1{}^s$ is bound; 2-methoxyethyl; phenyl or benzyl; carboxyalkyl of altogether 2 to 5 carbon atoms; alkoxycarbonylalkyl of 1 to 4 carbon atoms in the alkoxy group and of altogether 2 to 5 carbon atoms in the carbonylalkyl group thereof; or 4-pivaloylbenzyl;

either:

$R_2{}^s$ is: alkyl of 1 to 4 carbon atoms; or phenyl optionally monosubstituted in the 4 position by pivaloyl;

$R_3{}^s$ is: 4-pivaloylbenzoyl;

or:

$R_2{}^s$ is: 4 pivaloylphenyl and

$R_3{}^s$ is: alkylcarbonyl of altogether 2 to 5 carbon atoms; or benzoyl.

A further subgroup of compounds of formula I is the compounds of formula Ip

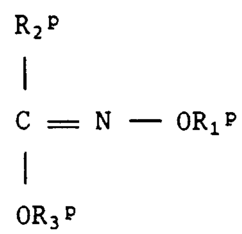

Ip

wherein

$R_1{}^p$ is: alkyl of 1 to 4 carbon atoms; alkenyl of 3 to 5 carbon atoms wherein the double bond is separated from the oxygen atom by at least 2 carbon atoms; mono-, di- or trihydroxyalkyl of 2 to 6 carbon atoms wherein the hydroxy groups are separated by at least 2 carbon atoms from the oxygen atom to which $R_1$ is bound; mono-, di- or trialkoxyalkyl of 1 to 4 carbon atoms in the alkoxy groups and of 2 to 6 carbon atoms in the alkylene group thereof, wherein the alkoxy groups are separated by at least 2 carbon atoms from the oxygen atom to which $R_1$ is bound; or phenyl or straight-chained phenylalkyl of 7 to 11 carbon atoms, both optionally mono- or independently disubstituted in the phenyl ring by halogen of atomic number of from 9 to 53, hydroxy, alkyl of 1 to 4 carbon atoms

or alkoxy of 1 to 4 carbon atoms; and

$R_2^p$ and $R_3^p$ have the significance indicated above for, respectively, $R_2$ and $R_3$.

A compound of the invention can be obtained by a process comprising

a) reacting a compound of formula II

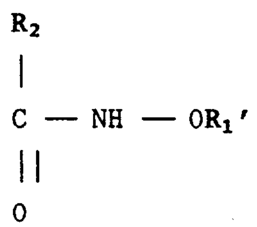

II

wherein

$R_1'$ has the significance indicated above for $R_1$ but with the hydroxy groups of any hydroxy alkyl or hydroxy phenyl substituent optionally in protected form and

$R_2$ is as defined above,

with a compound of formula III

$R_3 - X$ III

wherein

$R_3$ is as defined above and

X is a reactive group; or

b) for the preparation of a compound of formula Ia

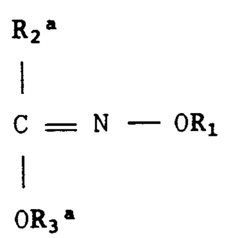

Ia

wherein

$R_1$ is as defined above;

$R_2^a$ is 4-pivaloylphenyl; and

$R_3^a$ is 4-pivaloylbenzoyl

reacting a compound of formula IVa

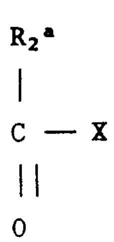

IVa

wherein $R_2^a$ and X are as defined above,

with a tertiary amine of formula V

$R_4R_5R_6N$ V

wherein $R_4$, $R_5$ and $R_6$ independently are lower alkyl or aryl, and a compound of formula VI

$H_2N - OR_1'$ VI

wherein $R_1'$ is as defined above,
and where indicated deprotecting any protected hydroxy alkyl or hydroxy phenyl substituent, and recovering the resultant compound of formula I in free form or salt or pharmaceutically acceptable and physiologically hydrolysable ester form as appropriate.

Process variant a) can be effected in accordance with known procedures. X preferably is halogen of atomic number of from 17 to 53, especially chlorine. In $R_1'$ any hydroxy alkyl or hydroxy phenyl substituent preferably is in protected form. The reaction preferably is effected in a non-polar solvent in the presence of a base. The base is e.g. an inorganic base, for example an alkali metal hydride, such as potassium hydride, or an alkali metal carbonate, such as potassium carbonate, preferably in a ratio of base to compound of formula II of from about 1:1 to 2:1 on a molar basis. The solvent can be any non-polar solvent, e.g. an aliphatic or aromatic hydrocarbon such as hexane, benzene or toluene, especially toluene; a halogenated hydrocarbon such as methylene chloride; an ether such as dioxane; and tetrahydrofuran. The temperature preferably is from about -40 °C to about 90 °C, especially from about -20 °C to about 60 °C, in particular from about -10 °C to about 30 °C. The reaction time is not critical and is about 1 to 16, preferably about 1 to 4, especially about 1 to 2 hours.

However, it has been found that, surprisingly, the above reaction, in addition to being time-saving, safer, and more economical, proceeds in much higher yields and leads to a much more pure compound of formula I when the base used is a hindered organic base such as a tertiary amine and in particular, triethylamine. The ratio of hindered base to compound of formula II is from about 1:1 to 5:1 on a molar basis, preferably of from about 1:1 to about 2:1.

The invention thus also includes the specific process variant for the preparation of a compound of formula I as defined above comprising reacting a compound of formula II as defined above with a compound of formula III as defined above in a non-polar solvent in the presence of a hindered organic base.

Process variant b) is effected in accordance with known procedures. It is advantageously effected by keeping the molar ratio of compound of formula IV to compound of formula VI at about 2:1. Preferably the reaction is effected with the compound of formula IV in a non-polar solvent with an aqueous solution of the tertiary amine of formula V. The reaction preferably is carried out in excess tertiary amine of formula V, in particular about 2 to 3 moles per mole of the compound of formula IV. Non-polar solvents which can be used are the same as in process variant a), preferably toluene. It is also preferred that the reaction be carried out with stirring at temperatures between about -10° to about 40 °C, starting at -10° to -5 °C while adding the reactants over a period of about 40 to 60 minutes, and then allowing the temperature to rise slowly to about 30° to 40 °C over a period of 1 to 2 hours. Aryl preferably is benzyl. Lower alkyl as a group $R_4$, $R_5$ and/or $R_6$ preferably is of 1 to 4 carbon atoms, it especially is methyl or ethyl, especially ethyl.

A compound of formula II can be prepared by reaction of a compound of formula IV

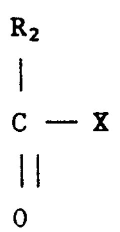

IV

wherein $R_2$ and X are as defined above,
with a compound of formula VI in the presence of a base, preferably an alkali metal base, preferably in a non-polar solvent.

A compound of formula II can alternatively be prepared by reaction of a compound of formula VII

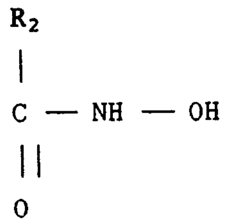

VII

wherein $R_2$ is as defined above,
with a compound of formula VIII

$R_1 - X$     VIII

wherein $R_1$ and X are as defined above,
in the presence of a base, preferably an alkali metal carbonate, preferably in an alcohol solvent, followed where indicated by appropriate protection.

It follows from the above preparation of the starting materials of formula II for process variant a) from compounds of formula IV, that process variant b), in which the compounds of formula IVa are used, is a one-pot version of process variant a) for a subgroup of compounds of formula I and thus belongs to the same inventive entity as process variant a).

Deprotection may be effected in conventional manner. The protecting group for the hydroxy alkyl substituents can be any hydroxy protecting group such as, for example, p-anisyldiphenylmethyl for mono hydroxy alkyl substituents, and acetonide-forming agents such as 2,2-dimethoxypropane for 1,2- and 1,3-diols. Protecting groups for the carboxyalkyl substituents can be any carboxy protecting group, preferably lower alkyl, especially tert-butyl. It will be appreciated that where the compound of formula I is desired in the form of a pharmaceutically acceptable and physiologically hydrolysable ester, the protected carboxyalkyl can be in the desired ester form.

The resultant compound of formula I can be isolated and purified in conventional manner, e.g. using evaporation and/or column chromatography and/or recrystallization. Recrystallization preferably is effected with a 5 % solution of toluene in methanol.

A compound of formula I can exist in the form of E or Z geometrical isomers. These can be prepared as such or readily separated and recovered by conventional techniques from isomeric mixtures. Such isomeric forms are included in the scope of this invention. The compounds obtained by the preferred variants of the present invention, i.e. using a hindered base, are predominantly in the form of a single, very probably the Z, isomer.

Insofar as its preparation is not specifically described, a compound used as a starting material is known or can be prepared in conventional manner starting from known compounds, e.g. as described in the Examples.

The closest prior art is represented by US-A-3,649,664 and GB-A-1,540,028. US-A-3,649,664 describes n-oxyimidic acid derivatives which have a phenyl group attached to the carbon atom doubly bound to nitrogen, and the acid OH group is acylated by e.g. alkylcarbonyl, benzylcarbonyl or phenylcarbonyl groups. The compounds of that document differ from those of the present invention essentially in that the phenyl groups are substituted by groups other than pivaloyl. The compounds of that document are useful as acaricides. GB-A-1,540,028 discloses i.a. N-oxyimidic acid derivatives which do not have aromatic groups bound to the carbon atom attached to nitrogen through a double bond (see table 3). These compounds are intermediates for the preparation of compounds having various pharmacological activity, e.g. anti-convulsive, analgesic, anthelmintic and anti-fungal activity.

The following Examples illustrate the invention. All temperatures are in degrees Centigrade.

**Example 1: 4-Pivaloylbenzoyl-N-methoxy-4-pivaloylbenzimidate**

[Formula I: $R_1$ = methyl; $R_2$ = 4-pivaloylphenyl;
$R_3$ = 4-pivaloylbenzoyl]
[Process variant a), using hindered organic base]

23.5 g **N-methoxy-4-pivaloylbenzamide**, 16.0 g **triethylamine** and 300 ml of toluene are stirred together for 15 minutes at 20°. The mixture is then cooled to -8° and 24.7 g **4-pivaloylbenzoyl chloride** in 200 ml of toluene are added at -8° to -5° over 30 minutes. The mixture is allowed to warm slowly to 22° over 30 minutes and is stirred at that temperature for an additional 30 minutes. It is then heated to 55° over 15 minutes and maintained at 45° to 50° for 1 hour. The mixture is then cooled to 22°, and 300 ml of water are added. After stirring for 10 minutes at 20° the water is discarded and the organic phase is washed two more times with 250 ml portions of water. The toluene phase is then evaporated to a white solid. The **title compound** is obtained (M.P. 104-104.8°; from toluene/heptane 1:2).

The starting material is obtained as follows:

a) 84 g **4-pivaloylbenzoic acid** in 210 ml of **thionyl chloride** are refluxed under nitrogen for 90 minutes. The excess thionyl chloride is then stripped off under reduced pressure. **4-Pivaloylbenzoyl chloride** (compound of formula IV) (M.P. 38-39°) is obtained.

b) 48.9 g **methoxyamine** hydrochloride (compound of formula VI) is added with stirring to a mixture of 660 ml of 1.5 N sodium hydroxide and 600 ml of tetrahydrofuran at 5°. After 30 minutes 87.8 g **4-pivaloylbenzoyl chloride** dissolved in 150 ml of tetrahydrofuran is added at 5° to 10°. The mixture is stirred at 5° for 30 minutes and the tetrahydrofuran is evaporated off to yield a slurry. The slurry is extracted four times with 500 ml of methylene chloride and the combined organic layers are washed with 5 % sodium bicarbonate and saturated sodium chloride. The organic layer is dried over magnesium sulfate and concentrated to about 300 ml. 600 ml of hexane is added to crystallize out **N-methoxy-4-pivaloylbenzamide** (compound of formula II) (M.P. 125-126.5°).

**Example 2: 4-Pivaloylbenzoyl-N-(2-hydroxyethoxy)-4-pivaloylbenzimidate**

[Formula I: $R_1$ = 2-hydroxyethyl; $R_2$ = 4-pivaloylphenyl; $R_3$ = 4-pivaloylbenzoyl]

[Process variant a), using potassium hydride; with deprotection]

1) To a solution of 5.25 g **N-(2-[p-anisyldiphenylmethyloxy]ethoxy)-4-pivaloylbenzamide** in 20 ml of tetrahydrofuran at 0° is added 700 mg hexane-washed **potassium hydride**, and after 20 minutes, 2.3 g **4-pivaloylbenzoyl chloride** are added. This mixture is allowed to warm to room temperature and stirred for one hour, then it is poured into 50 ml of saturated ammonium chloride and extracted twice with 50 ml of t-butyl methyl ether. The combined organic layers are washed with 50 ml of water and 50 ml of brine, then dried over magnesium sulfate and evaporated to dryness.

2) **Deprotection**: the crude product is dissolved in 30 ml of tetrahydrofuran at 0° and 0.75 ml of 2 N hydrochloric acid is added. This mixture is stored at 0° for 60 hours. After deprotection is complete, the mixture is poured into 100 ml of 10 % sodium bicarbonate and extracted twice with 100 ml of ethyl acetate. The combined organic layers are washed with 50 ml of water and 50 ml of brine, dried over magnesium sulfate and concentrated by evaporation. The product is purified by chromatography using 1:1 hexane/ethyl acetate as the eluant, and recrystallized from 3:1 hexane/chloroform. The **title compound** is obtained (M.P. 106°).

The starting material is obtained as follows:

a) To a solution of 4.0 g **N-hydroxy-4-pivaloylbenzamide** (compound of formula VII) in 10 ml of ethanol is added a solution of 725 mg sodium hydroxide in 1 ml of water. To this is added 1.67 ml of **2-bromoethanol** (compound of formula VIII), and the resulting mixture is heated at 55° for 24 hours. The mixture is cooled to 20°, concentrated, and extracted 3 times with 10 ml of chloroform. The combined extracts are washed with 15 ml of water and then 15 ml of brine, dried over magnesium sulfate and concentrated. The residue is recrystallized from methylene chloride/ hexane 1:4. **N-(2-Hydroxyethoxy)-4-pivaloylbenzamide** is obtained.

b) **Protection**: to a solution of 2.8 g product of step a) above in 5 ml of methylene chloride and 3 ml of pyridine at 0° is added 3.92 g of **p-anisylchlorodiphenylmethane**. The mixture is warmed to room temperature over two hours and then diluted with 40 ml of methylene chloride. This solution is washed with 50 ml of 10 % sodium bicarbonate and 30 ml of brine, dried over magnesium sulfate and concentrated by evaporation. The residue is purified by column chromatography using 5:1 hexane/ethyl acetate as the eluant. The protected product, **N-(2-[p-anisyldiphenylmethyloxy]ethoxy)-4-pivaloylbenzamide** (compound of formula II) is obtained.

**Example 3: 4-Pivaloylbenzoyl-N-methoxy-4-pivaloylbenzimidate**

[Formula I: $R_1$, $R_2$, $R_3$ = as for Example 1]
[Process variant b); one-pot]

At room temperature, 101.3 g (0.373 mol) of 30.8 % aqueous **methoxyamine** (compound of formula VI) hydrochloride solution is diluted with 500 ml of deionized water. To this solution is added 165.3 g (1.636 mol) of **triethylamine** (compound of formula V) at a rate such that the internal temperature remains between 22° and 30° over a period of 5 to 10 minutes. This solution is cooled to an internal temperature of -7° to -10°; and then slowly with efficient stirring, a solution of **4-pivaloylbenzoyl chloride** (compound of formula IVa; prepared from 150 g [0.725 mol] of 4-pivaloylbenzoic acid and 180 ml [2.47 mol] of thionyl chloride) in 1 l of dry toluene is added over a period of 45 to 60 minutes while maintaining the internal temperature between -5° and -7°. The reaction mixture is allowed to warm to 22° and then warmed to 33° to 35° and maintained at this temperature for an additional 20 minutes. The layers are separated, the organic layer is washed with saturated aqueous sodium carbonate solution and then with three portions of deionized water. The toluene layer is filtered and the organic phase stripped under vacuum (20-30 torr) at 55° to 60° to yield a yellow oil, which is dissolved at 50° to 55° in a mixture of 1100 ml of methyl alcohol and 57 ml of toluene. The solution is heated at reflux and then cooled to 22° with stirring. Agitation is continued at a temperature of 22° to 25°, and the solids formed are collected by filtration. The filter cake is washed with 60 ml of cold methanol and the solids are dried in vacuo (25 to 30 torr) at 60° to 70° to yield the **title compound** (M.P. 104.5°).

**Example 3a: 4-Pivaloylbenzoyl-N-carboxymethoxy-4-pivaloylbenzimidate**

[Formula I: $R_1$ = carboxymethyl; $R_2$ = 4-pivaloylphenyl;
$R_3$ = 4-pivaloylbenzoyl]
[Deprotection]

A solution of 2.3 g of **4-pivaloylbenzoyl-N-(t-butoxycarbonylmethyl)-4-pivaloylbenzimidate** - (compound of Example 25) in 50 ml of 5 % trifluoroacetic acid in chloroform is allowed to stand at room temperature for 3½ hours. The reaction mixture is evaporated under reduced pressure, and the residue is crystallized from 1:4 ether/hexane. The **title compound** is obtained (M.P. 72-74°).

The following compounds of formula I are obtained in analogous manner from corresponding starting material:

| Example No. | $R_1$ | $R_2$ | $R_3$ | Process | Analogous to Ex. No. | M.P. |
|---|---|---|---|---|---|---|
| 4 | 2,3-di-OH-propyl | 4-pivaloylphenyl | 4-pivaloylbenzoyl | a); b) | 1[2]; 2[1]; 3[2] | 64° |
| 5 | 2-OH-ethyl | 4-pivaloylphenyl | 4-pivaloylbenzoyl | a); b) | 1[3]; 3 | 106° |
| 6 | methyl | 4-pivaloylphenyl | 4-pivaloylbenzoyl | a) | 2 | 103-104° |
| 7 | isopropyl | 4-pivaloylphenyl | 4-pivaloylbenzoyl | a); b) | 1; 2; 3 | 76° |
| 8 | phenyl | 4-pivaloylphenyl | 4-pivaloylbenzoyl | a); b) | 1; 2; 3 | 143° |
| 9 | benzyl | 4-pivaloylphenyl | 4-pivaloylbenzoyl | a); b) | 1; 2; 3 | 126° |
| 10 | allyl | 4-pivaloylphenyl | 4-pivaloylbenzoyl | a); b) | 1; 2; 3 | 76° |
| 11 | methyl | phenyl | 4-pivaloylbenzoyl | a) | 1; 2 | 105-107° |
| 12 | methyl | t-butyl | 4-pivaloylbenzoyl | a) | 1; 2 | 60-62° |
| 13 | methyl | methyl | 4-pivaloylbenzoyl | a) | 1; 2 | oil; NMR[7] |
| 14 | methyl | 4-pivaloylphenyl | pivaloyl | a); b) | 1; 2; 3 | 48-50° |
| 15 | methyl | 4-pivaloylphenyl | benzoyl | a) | 1; 2 | 63-65° |
| 16 | methyl | 4-pivaloylphenyl | acetyl | a) | 1; 2 | 32-34° |
| 17 | ethyl | 4-pivaloylphenyl | 4-pivaloylbenzoyl | a); b) | 1; 2; 3 | 68° |
| 18 | t-butyl | 4-pivaloylphenyl | 4-pivaloylbenzoyl | a); b) | 1; 2; 3 | 102° |
| 19 | 2-MeO-ethyl | 4-pivaloylphenyl | 4-pivaloylbenzoyl | a); b) | 1; 2; 3 | NMR[8] |
| 20 | methyl | 4-OH-phenyl | 4-pivaloylbenzoyl | a) | 1; 2 | |
| 21 | methyl | 4-Cl-phenyl | 4-pivaloylbenzoyl | a) | 1; 2 | |
| 22 | methyl | 4-Me-phenyl | 4-pivaloylbenzoyl | a) | 1; 2 | |
| 23 | methyl | 4-MeO-phenyl | 4-pivaloylbenzoyl | a) | 1; 2 | |
| 24 | methyl | 4-pivaloylphenyl | benzyl | a) | 1; 2 | |

| Example No. | $R_1$ | $R_2$ | $R_3$ | Process | Analogous to Ex. No. | M.P. |
|---|---|---|---|---|---|---|
| 25[4)] | **t-butoxycarbonylmethyl** | **4-pivaloylphenyl** | **4-pivaloylbenzoyl** | a);b) | 1; 2; 3 | 112-115° |
| 26[4)] | **t-butoxycarbonylpropyl** | **4-pivaloylphenyl** | **4-pivaloylbenzoyl** | a);b) | 1; 2; 3 | 65-67° |
| 26a[4)] | **t-butoxycarbonylprop-2-yl** | **4-pivaloylphenyl** | **4-pivaloylbenzoyl** | a);b) | 1; 2; 3 | oil; NMR[9)] |
| 27 | **carboxypropyl** | **4-pivaloylphenyl** | **4-pivaloylbenzoyl** | deprot.;a);b) | 3a[5)]; 1; 2; 3 | 125-126° |
| 28 | **2-carboxy-2-propyl** | **4-pivaloylphenyl** | **4-pivaloylbenzoyl** | deprot.;a);b) | 3a[6)]; 1; 2; 3 | 95-97° |
| 29 | **4-pivaloylbenzyl** | **4-pivaloylphenyl** | **4-pivaloylbenzoyl** | a);b) | 1; 2; 3 | 78-80° |
| 30 | **carboxymethyl** | **4-pivaloylphenyl** | **4-pivaloylbenzoyl** | a);b) | 1; 2; 3 | 72-74° |

[1)]Using N-(2,3-dihydroxypropoxy)-4-pivaloylbenzamide instead of N-(2-hydroxyethyloxy)-4-pivaloylbenzamide, and 2,3-dimethoxypropane instead of p-anisylchlorodiphenylmethane, i.e. the compound of formula II has $R_1'$ = (2,2-dimethyl-1,3-dioxolan-4-yl)methyl;

[2)]With protection and deprotection as in footnote 1);

[3)]With protection and deprotection as in Example 2;

[4)]Using N-hydroxy-4-pivaloylbenzamide (compound of formula VII; prepared as described under b) in Example 1, using an equivalent amount of hydroxylamine in place of methoxyamine) to prepare the corresponding compound of formula II, e.g. N-(t-butoxycarbonylmethoxy)-4-pivaloylbenzamide (M.P. 87-88°);

[5)]Starting from the compound of Example 26;

[6)]Starting from the compound of Example 26a;

7) $^1$H-NMR ($CDCl_3$): 1.35 (9H,s); 2.17 (3H,s); 3.84 (3H,s); 7.71 (2H,d); 8.16 (2H,d);

8) $^1$H-NMR ($CDCl_3$): 1.32 (9H,s); 1.33 (9H,s); 3.32 (3H,s); 3.68 (2H,t,J=7Hz); 4.32 (t,J=7Hz); 7.63-7.85 (6H,m); 8.21 (2H,m);

9) $^1$H-NMR ($CDCl_3$): 1.33 (9H,s); 1.36 (9H,s); 1.45 (9H,s); 1.50 (6H,s); 7.73 (6H,m); 8.23 (2H,m).

The compounds of formula I possess interesting pharmacological activity. They are indicated for use as pharmaceuticals.

In particular, they possess anti-diabetic and hypoglycemic activity.

Anti-diabetic activity can be determined e.g. in the chronic hypoglycemic screen test in male Sprague-Dawley rats given 1 mg/kg to 100 mg/kg per day of drug orally. The rats, 2 to 3 months of age, weighing

200 to 220 grams, are kept in a room at a controlled ambient temperature of 22° C and a 12/12 hour light/dark cycle for one week before and during testing. In the chronic screen test, the rats are fed a high fat diet ad libitum. At fed state, 40 mg of streptozotocin/kg body weight are injected via the tail vain. One week later, those rats are considered to be diabetic which have fed blood glucose of greater than 200 mg/dl and, following an overnight fast, when given an oral glucose tolerance test have blood glucose of 41 to 80 mg/dl 3 hours after the test. Blood glucose is determined with a YSI Glucose Analyzer.

On Day 1, food is removed from rats at 9:00 a.m.; and after an initial blood glucose reading is taken via the tail vein, vehicle (control) or compound (9 rats/treatment) is administered orally. Six hours later blood glucose level is measured and immediately thereafter the rats are refed. The same rats are given either vehicle or drug once a day for 11 consecutive days. Blood glucose is then determined after a 6-hour fast post dosing on days 4, 8, and 11. The $ED_{50}$ value is the amount of compound required to produce a 50 % reduction on day 11 of the average increase in blood glucose level induced by streptozotocin.

The compounds of formula I are active in the above test at a dosage of from about 1 mg/kg to about 100 mg/kg.

Hypolipidemic activity can be determined e.g. in the above Sprague-Dawley rats following the chronic hypoglycemia determination. After the glucose sample is removed on day 11, the rats are sacrificed and blood serum is collected and adjusted to a density of 1.06 g/ml with sodium chloride. The density-adjusted blood serum is centrifuged at 42,000 rpm in a Beckmann 42.2 Ti rotor at 20° C for 2.5 hours in a Beckmann L8-M ultracentrifuge. 95 µl is fractionated from the top of the centrifuged mixture (LDL fraction), leaving 80 µl in the bottom (HDL fraction). Cholesterol is determined with the Sigma Diagnostic kit for the enzymatic determination of cholesterol, procedure No. 352, modified for use with 96 well microliter plates. 20 µl of calibrator, standard, or sample are mixed with 200 µl aliquots of enzyme reagent in the 96 wells and incubated at ambient temperature for 15 minutes. Total cholesterol and HDL are determined by absorbance measurement at 500 nm with a colorimetric micrometer plate reader. LDL is determined by subtracting HDL from total cholesterol. Total triglycerides in the blood serum are determined using the Boehringer Mannheim Diagnostic Reagents set R Triglycerides-GB kit modified for microtiter plate assays as follows: the contents of bottle 2 (enzymes) and bottle 3 (lipase + 4-aminoantipyrine) are each diluted to 8 ml with buffer (bottle 1) and stored in 2 ml aliquots in cryovials at -90° C. 10 ml of buffer is added to a 2 ml aliquot of diluted enzymes and to a 2 ml aliquot of diluted lipase + 4-aminoantipyrine solution to form working solutions 1 and 2, respectively. 100 µl of working solution 1 and 20 µl of dilute blood serum (1:1 blood serum: saline) are added to each well of a ninety-six microtiter plate and mixed and incubated at 20-25° C for at least 5 minutes. 100 µl of working solution 2 is added to each well, and the contents of each well are mixed and again incubated at 20-25° C for at least 5 minutes. Absorbance is measured at 500 nm and total triglyceride contents in mg/dl of blood serum is calculated by comparing the absorbance with the absorbance of known samples.

The compounds of formula I are active in the above test at a dosage of from about 1 mg/kg to about 100 mg/kg.

The compounds of formula I are therefore indicated for use in the treatment of diabetes and in lowering blood cholesterol and triglyceride level. The dosage to be employed will vary depending on the particular compound employed, the mode of administration and severity of the condition being treated. However, in general, satisfactory results are obtained when the compounds of formula (I) are administered at a daily dosage of from about 1 mg/kg to about 100 mg/kg of animal body weight, optionally given in divided doses two to four times a day, or in sustained release form. For the larger mammals, for example primates such as humans, the total daily dosage is from about 5 mg to about 500 mg per day. Unit dosage forms comprise from about 1 mg to about 500 mg of the active compound in admixture with a solid or liquid pharmaceutically acceptable carrier or diluent. The compounds of the invention may be administered in a manner similar to known standards for the above uses. The suitable daily dosage for a particular compound will depend on a number of factors, such as its relative potency of activity.

It has been determined that the preferred compound of the invention, 4-pivaloylbenzoyl-N-methoxy-4-pivaloylbenzimidate (Examples 1, 3 and 6), has an $ED_{50}$ of 28 mg/kg in the chronic hypoglycemia test. An indicated daily dosage for this compound is from about 100 mg to about 500 mg, preferably from about 150 mg to about 250 mg p.o.

For the above uses the compounds of formula I may be administered orally or parenterally as such or admixed with conventional pharmaceutical carriers. They may be administered orally in such forms as tablets, dispersible powders, granules, capsules, syrups and elixirs, and parenterally as solutions or emulsions. These pharmaceutical preparations may contain up to about 90 % of the active ingredient in combination with the carrier or adjuvant.

Capsules containing the ingredients indicated below may be prepared by conventional techniques and are indicated for use in the above indications at a dose of one or two capsules, two to four times a day:

| Ingredient | Weight (mg) |
|---|---|
| Compound of Example 1 | 250 |
| Lactose | 445 |
| Colloidal silicon | 50 |
| Stearic acid | 5 |
| | total 750 mg |

The invention thus also concerns a pharmaceutical composition comprising a compound of formula I in free form or pharmaceutically acceptable salt or pharmaceutically acceptable and physiologically hydrolysable ester form as appropriate, together with a pharmaceutically acceptable carrier or diluent.

It further comprises a such compound of formula I for use as a pharmaceutical, particularly as an antidiabetic and blood cholesterol and triglyceride level lowering agent.

It further comprises, a process for the preparation of a pharmaceutical composition which comprises mixing a such compound of formula I with a pharmaceutically acceptable carrier or diluent.

It further comprises the use of a such compound of formula I for the manufacture of a medicament, particularly for the manufacture of a medicament for the treatment of diabetes and for lowering blood cholesterol and triglyceride level.

The compound of Examples 1, 3 and 6 is preferred. It can also be named N-methoxy-4-pivaloylbenzimidic acid, 4-pivaloylbenzoic acid anhydride.

## Claims

**Claims for the following Contracting States : DE, GB, FR, IT, NL, SE, LI, CH, BE, AT, LU, DK**

**1.** A compound of formula I

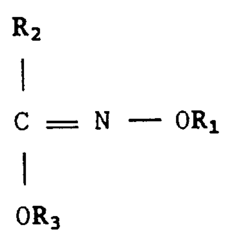

I

wherein

$R_1$ is: alkyl of 1 to 6 carbon atoms; alkenyl of 3 to 5 carbon atoms wherein the double bond is separated from the oxygen atom by at least 2 carbon atoms; mono- or polyhydroxyalkyl of 2 to 8 carbon atoms having up to 7 hydroxy groups, wherein the hydroxy groups are separated by at least 2 carbon atoms from the oxygen atom to which $R_1$ is bound; mono- or polyalkoxyalkyl of 1 to 4 carbon atoms in the alkoxy groups and of 2 to 8 carbon atoms in the alkylene group thereof, having up to 7 alkoxy groups, wherein the alkoxy groups are separated by at least 2 carbon atoms from the oxygen atom to which $R_1$ is bound; phenyl or straight-chained phenylalkyl of 7 to 11 carbon atoms, both optionally mono- or independently disubstituted in the phenyl ring by halogen of atomic number of from 9 to 53, hydroxy, alkyl of 1 to 4 carbon atoms or alkoxy of 1 to 4 carbon atoms; carboxyalkyl of altogether 2 to 6 carbon atoms; alkoxycarbonylalkyl of 1 to 4 carbon atoms in the alkoxy group and of altogether 2 to 6 carbon atoms in the carbonylalkyl group thereof; or 4-pivaloylbenzyl;

either:

$R_2$ is: alkyl of 1 to 4 carbon atoms; phenyl or straight-chained phenylalkyl of 7 to 11 carbon atoms, both either optionally mono- or independently disubstituted in the phenyl ring by halogen of atomic number of from 9 to 53, hydroxy, alkyl of 1 to 4 carbon atoms or alkoxy of 1 to 4 carbon atoms, or monosubstituted in the 4 position of the phenyl ring by pivaloyl;

and

$R_3$ is: 4-pivaloylbenzoyl;

or:

$R_2$ is: 4-pivaloylphenyl and

$R_3$ is: alkyl of 1 to 4 carbon atoms; straight-chained phenylalkyl of 7 to 11 carbon atoms, either optionally mono- or independently disubstituted in the phenyl ring by halogen of atomic number of from 9 to 53, hydroxy, alkyl of 1 to 4 carbon atoms or alkoxy of 1 to 4 carbon atoms, or monosubstituted in the 4 position of the phenyl ring by pivaloyl; alkylcarbonyl of altogether 2 to 5 carbon atoms; or benzoyl optionally mono- or independently disubstituted by halogen of atomic number of from 9 to 53, hydroxy, alkyl of 1 to 4 carbon atoms or alkoxy of 1 to 4 carbon atoms;

in free form or salt or pharmaceutically acceptable and physiologically hydrolysable ester form as appropriate.

**2.** A compound according to claim 1 of formula Is

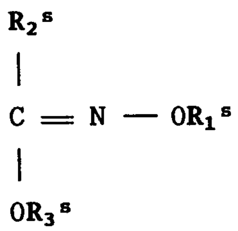

Is

wherein

$R_1{}^s$ is: alkyl of 1 to 4 carbon atoms; allyl; mono- or dihydroxyalkyl of 2 to 4 carbon atoms wherein the hydroxy groups are separated by at least 2 carbon atoms from the oxygen atom to which $R_1{}^s$ is bound; 2-methoxyethyl; phenyl or benzyl; carboxyalkyl of altogether 2 to 5 carbon atoms; alkoxycarbonylalkyl of 1 to 4 carbon atoms in the alkoxy group and of altogether 2 to 5 carbon atoms in the carbonylalkyl group thereof; or 4-pivaloylbenzyl;

either:

$R_2{}^s$ is: alkyl of 1 to 4 carbon atoms; or phenyl optionally monosubstituted in the 4 position by pivaloyl;

$R_3{}^s$ is: 4-pivaloylbenzoyl;

or:

$R_2{}^s$ is: 4 pivaloylphenyl and

$R_3{}^s$ is: alkylcarbonyl of altogether 2 to 5 carbon atoms; or benzoyl.

**3.** A compound according to claim 1 of formula Ip

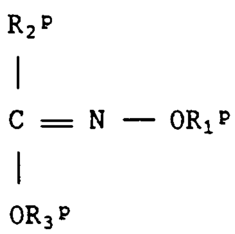

Ip

wherein

$R_1{}^p$ is: alkyl of 1 to 4 carbon atoms; alkenyl of 3 to 5 carbon atoms wherein the double bond is separated from the oxygen atom by at least 2 carbon atoms; mono-, di- or trihydroxyalkyl of 2 to 6 carbon atoms wherein the hydroxy groups are separated by at least 2 carbon atoms from the oxygen atom to which $R_1$ is bound; mono-, di- or trialkoxyalkyl of 1 to 4 carbon atoms in the alkoxy groups and of 2 to 6 carbon

atoms in the alkylene group thereof, wherein the alkoxy groups are separated by at least 2 carbon atoms from the oxygen atom to which $R_1$ is bound; or phenyl or straight-chained phenylalkyl of 7 to 11 carbon atoms, both optionally mono- or independently disubstituted in the phenyl ring by halogen of atomic number of from 9 to 53, hydroxy, alkyl of 1 to 4 carbon atoms or alkoxy of 1 to 4 carbon atoms; and

$R_2{}^p$ and $R_3{}^p$ have the significance indicated above for, respectively, $R_2$ and $R_3$.

**4.** The compound according to claim 1 which is 4-pivaloylbenzoyl-N-methoxy-4-pivaloylbenzimidate.

**5.** The compound according to claim 1 which is 4-pivaloylbenzoyl-N-(2-hydroxyethoxy)-4-pivaloylbenzimidate or 4-pivaloylbenzoyl-N-carboxymethoxy-4-pivaloylbenzimidate, or is of formula I wherein $R_1$, $R_2$ and $R_3$ respectively are
either 2,3-di-OH-propyl, 4-pivaloylphenyl and 4-pivaloylbenzoyl,
or 2-OH-ethyl, 4-pivaloylphenyl and 4-pivaloylbenzoyl,
or isopropyl, 4-pivaloylphenyl and 4-pivaloylbenzoyl,
or phenyl, 4-pivaloylphenyl and 4-pivaloylbenzoyl,
or benzyl, 4-pivaloylphenyl and 4-pivaloylbenzoyl,
or allyl, 4-pivaloylphenyl and 4-pivaloylbenzoyl,
or methyl, phenyl and 4-pivaloylbenzoyl,
or methyl, t-butyl and 4-pivaloylbenzoyl,
or methyl, methyl and 4-pivaloylbenzoyl,
or methyl, 4-pivaloylphenyl and pivaloyl,
or methyl, 4-pivaloylphenyl and benzoyl,
or methyl, 4-pivaloylphenyl and acetyl,
or ethyl, 4-pivaloylphenyl and 4-pivaloylbenzoyl,
or t-butyl, 4-pivaloylphenyl and 4-pivaloylbenzoyl,
or 2-MeO-ethyl, 4-pivaloylphenyl and 4-pivaloylbenzoyl,
or methyl, 4-OH-phenyl and 4-pivaloylbenzoyl,
or methyl, 4-Cl-phenyl and 4-pivaloylbenzoyl,
or methyl, 4-Me-phenyl and 4-pivaloylbenzoyl,
or methyl, 4-MeO-phenyl and 4-pivaloylbenzoyl,
or methyl, 4-pivaloylphenyl and benzyl,
or t-butoxycarbonylmethyl, 4-pivaloylphenyl and 4-pivaloylbenzoyl,
or t-butoxycarbonylpropyl, 4-pivaloylphenyl and 4-pivaloylbenzoyl,
or t-butoxycarbonylprop-2-yl, 4-pivaloylphenyl and 4-pivaloylbenzoyl,
or carboxypropyl, 4-pivaloylphenyl and 4-pivaloylbenzoyl,
or 2-carboxy-2-propyl, 4-pivaloylphenyl and 4-pivaloylbenzoyl,
or 4-pivaloylbenzyl, 4-pivaloylphenyl and 4-pivaloylbenzoyl,
in free form or salt or pharmaceutically acceptable and physiologically hydrolysable ester form as appropriate.

**6.** A process for the preparation of a compound according to claim 1 comprising
a) reacting a compound of formula II

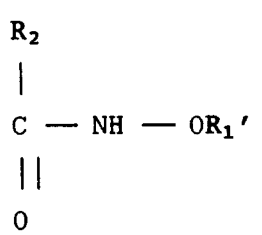

II

wherein

$R_1'$ has the significance indicated in claim 1 for $R_1$ but with the hydroxy groups of any hydroxy alkyl or hydroxy phenyl substituent optionally in protected form and

$R_2$ is as defined in claim 1,

with a compound of formula III

$R_3 - X$ III

wherein

$R_3$ is as defined in claim 1, and

X is a reactive group; or

b) for the preparation of a compound of formula Ia

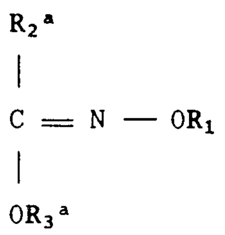

Ia

wherein

$R_1$ is as defined in claim 1;

$R_2{}^a$ is 4-pivaloylphenyl; and

$R_3{}^a$ is 4-pivaloylbenzoyl

reacting a compound of formula IVa

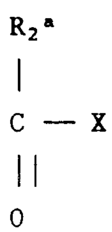

IVa

wherein $R_2{}^a$ and X are as defined in this claim,
with a tertiary amine of formula V

$R_4 R_5 R_6 N$ V

wherein $R_4$, $R_5$ and $R_6$ independently are lower alkyl or aryl, and
a compound of formula VI

$H_2N - OR_1{}'$ VI

wherein $R_1{}'$ is as defined in this claim,
and where indicated deprotecting any protected hydroxy alkyl or hydroxy phenyl substituent, and recovering the resultant compound of formula I in free form or salt or pharmaceutically acceptable and physiologically hydrolysable ester form as appropriate.

**7.** A pharmaceutical composition comprising a compound according to claim 1 in free form or pharmaceutically acceptable salt or pharmaceutically and physiologically hydrolysable ester form as appropriate, together with a pharmaceutically acceptable carrier or diluent.

**8.** A compound as defined in claim 7 for use as an anti-diabetic and blood cholesterol and triglyceride level lowering agent.

**9.** Use of a compound according to claim 7 for the manufacture of a medicament for the treatment of diabetes and for lowering blood cholesterol and triglyceride level.

**Claims for the following Contracting States : ES, GR**

**1.** A process for the preparation of a compound of formula I

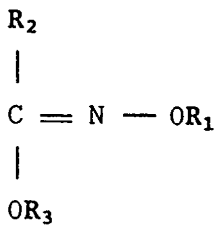

I

wherein

$R_1$ is: alkyl of 1 to 6 carbon atoms; alkenyl of 3 to 5 carbon atoms wherein the double bond is separated from the oxygen atom by at least 2 carbon atoms; mono- or polyhydroxyalkyl of 2 to 8 carbon atoms having up to 7 hydroxy groups, wherein the hydroxy groups are separated by at least 2 carbon atoms from the oxygen atom to which $R_1$ is bound; mono- or polyalkoxyalkyl of 1 to 4 carbon atoms in the alkoxy groups and of 2 to 8 carbon atoms in the alkylene group thereof having up to 7 alkoxy groups, wherein the alkoxy groups are separated by at least 2 carbon atoms from the oxygen atom to which $R_1$ is bound; phenyl or straight-chained phenylalkyl of 7 to 11 carbon atoms, both optionally mono- or independently disubstituted in the phenyl ring by halogen of atomic number of from 9 to 53, hydroxy, alkyl of 1 to 4 carbon atoms or alkoxy of 1 to 4 carbon atoms; carboxyalkyl of altogether 2 to 6 carbon atoms; alkoxycarbonylalkyl of 1 to 4 carbon atoms in the alkoxy group and of altogether 2 to 6 carbon atoms in the carbonylalkyl group thereof; or 4-pivaloylbenzyl;

either:

$R_2$ is: alkyl of 1 to 4 carbon atoms; phenyl or straight-chained phenylalkyl of 7 to 11 carbon atoms, both either optionally mono- or independently disubstituted in the phenyl ring by halogen of atomic number of from 9 to 53, hydroxy, alkyl of 1 to 4 carbon atoms or alkoxy of 1 to 4 carbon atoms, or monosubstituted in the 4 position of the phenyl ring by pivaloyl; and

$R_3$ is: 4-pivaloylbenzoyl;

or:

$R_2$ is: 4-pivaloylphenyl and

$R_3$ is: alkyl of 1 to 4 carbon atoms; straight-chained phenylalkyl of 7 to 11 carbon atoms, either optionally mono- or independently disubstituted in the phenyl ring by halogen of atomic number of from 9 to 53, hydroxy, alkyl of 1 to 4 carbon atoms or alkoxy of 1 to 4 carbon atoms, or monosubstituted in the 4 position of the phenyl ring by pivaloyl; alkylcarbonyl of altogether 2 to 5 carbon atoms; or benzoyl optionally mono- or independently disubstituted by halogen of atomic number of from 9 to 53, hydroxy, alkyl of 1 to 4 carbon atoms or alkoxy of 1 to 4 carbon atoms;

in free form or salt or pharmaceutically acceptable and physiologically hydrolysable ester form as appropriate,

comprising

a) reacting a compound of formula II

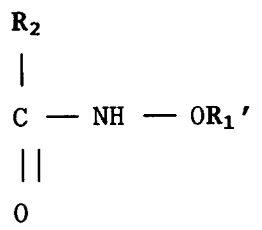

II

wherein

$R_1$' has the significance indicated in this claim for $R_1$ but with the hydroxy groups of any hydroxy alkyl or hydroxy phenyl substituent optionally in protected form and

$R_2$ is as defined in this claim,

with a compound of formula III

$R_3 - X$ III

wherein

$R_3$ is as defined in this claim and

X is a reactive group; or

b) for the preparation of a compound of formula Ia

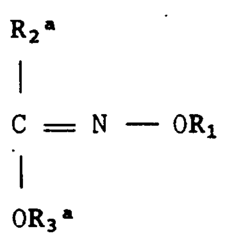

Ia

wherein

$R_1$' is as defined in this claim;

$R_2{}^a$ is 4-pivaloylphenyl; and

$R_3{}^a$ is 4-pivaloylbenzoyl

reacting a compound of formula IVa

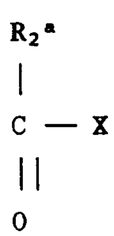

IVa

wherein $R_2{}^a$ and X are as defined in this claim,

with a tertiary amine of formula V

$R_4R_5R_6N$ V

wherein $R_4$, $R_5$ and $R_6$ independently are lower alkyl or aryl, and

a compound of formula VI

$H_2N - OR_1$' VI

wherein $R_1$' is as defined in this claim,
and where indicated deprotecting any protected hydroxy alkyl or hydroxy phenyl substituent, and recovering the resultant compound of formula I in free form or salt or pharmaceutically acceptable and physiologically hydrolysable ester form as appropriate.

**2.** A process according to claim 1 for the preparation of the compound of formula I which is 4-pivaloylbenzoyl-N-methoxy-4-pivaloylbenzimidate comprising
a) reacting the compound of formula II wherein $R_1$' is methyl and $R_2$ is 4-pivaloylphenyl, with a compound of formula III wherein $R_3$ is 4-pivaloylbenzoyl and X is as defined in claim 1 or
b) reacting a compound of formula IVa wherein $R_2{}^a$ is 4-pivaloylphenyl and X is as defined in claim 1, with a tertiary amine of formula V wherein $R_4$, $R_5$ and $R_6$ are as defined in claim 1, and a compound of formula VI wherein $R_1$' is methyl.

**3.** A process according to claim 2 comprising
a) reacting N-methoxy-4-pivaloylbenzamide with 4-pivaloylbenzoyl chloride or
b) reacting 4-pivaloylbenzoyl chloride with triethylamine and methoxyamine.

**4.** A process according to claim 1 comprising the use of a hindered organic base.

**5.** A process according to claim 4 comprising the use of triethylamine.

**6.** A process for the preparation of a pharmaceutical composition comprising mixing a compound of formula I as defined in claim 1 in free form or pharmaceutically acceptable salt or pharmaceutically acceptable and physiologically hydrolysable ester form as appropriate, together with a pharmaceutically acceptable carrier or diluent.

**Patentansprüche**
**Patentansprüche für folgende Vertragsstaaten : DE, GB, FR, IT, NL, SE, LI, CH, BE, AT, LU, DK**

**1.** Verbindung der Formel I

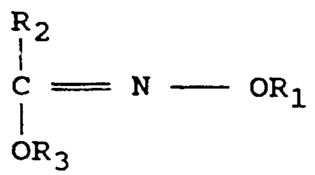

I

worin
$R_1$ ein Alkylrest mit 1 bis 6 Kohlenstoffatomen; ein Alkenylrest mit 3 bis 5 Kohlenstoffatomen, worin die Doppelbindung von dem Sauerstoffatom durch mindestens 2 Kohlenstoffatome getrennt ist; ein Mono- oder Polyhydroxyalkylrest mit 2 bis 8 Kohlenstoffatomen mit bis zu 7 Hydroxygruppen, worin die Hydroxygruppen durch mindestens 2 Kohlenstoffatome von dem Sauerstoff, an den $R_1$ gebunden ist, getrennt sind; ein Mono- oder Polyalkoxyalkylrest mit 1 bis 4 Kohlenstoffatomen in den Alkoxygruppen und 2 bis 8 Kohlenstoffatomen in der Alkylengruppe mit bis zu 7 Alkoxygruppen, worin die Alkoxygruppen durch mindestens 2 Kohlenstoffatome von dem Sauerstoff, an den $R_1$ gebunden ist, getrennt sind; ein Phenyl- oder geradkettiger Phenylalkylrest mit 7 bis 11 Kohlenstoffatomen, der gegebenenfalls jeweils am Phenylring mono- oder unabhängig disubstitutiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen; ein Carboxyalkylrest mit insgesamt 2 bis 6 Kohlenstoffatomen; ein Alkoxycarbonylalkylrest mit 1 bis 4 Kohlenstoffatomen in der Alkoxygruppe und insgesamt 2 bis 6 Kohlenstoffatomen in der Carbonylalkylgruppe; oder ein 4-Pivaloylbenzylrest ist;
entweder:
$R_2$ ein Alkylrest mit 1 bis 4 Kohlenstoffatomen; ein Phenyl- oder geradkettiger Phenylalkylrest mit 7 bis 11 Kohlenstoffatomen, der gegebenenfalls jeweils am Phenylring mono- oder

unabhängig disubstituiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen oder in Position 4 des Phenylrings mit einem Pivaloylrest monosubstituiert ist, ist; und

$R_3$ ein 4-Pivaloylbenzoylrest ist;

oder

$R_2$ ein 4-Pivaloylphenylrest ist; und

$R_3$ ein Alkylrest mit 1 bis 4 Kohlenstoffatomen; ein geradkettiger Phenylalkylrest mit 7 bis 11 Kohlenstoffatomen, der am Phenylring gegebenenfalls entweder mono- oder unabhängig disubstituiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen oder in Position 4 des Phenylrings mit einem Pivaloylrest monosubstituiert ist; ein Alkylcarbonylrest mit insgesamt 2 bis 5 Kohlenstoffatomen; oder ein Benzoylrest, der gegebenenfalls mono- oder unabhängig disubstituiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen, ist;

je nachdem in freier Form oder Salzform oder in Form eines pharmazeutisch annehmbaren und physiologisch hydrolysierbaren Esters.

**2.** Verbindung nach Anspruch 1 der Formel Is

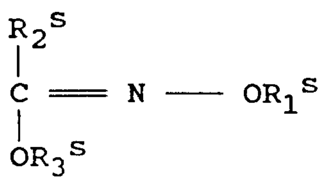

Is

worin

$R_1^s$ ein Alkylrest mit 1 bis 4 Kohlenstoffatomen; ein Allylrest; ein Mono- oder Dihydroxyalkylrest mit 2 bis 4 Kohlenstoffatomen, worin die Hydroxygruppen durch mindestens 2 Kohlenstoffatome von dem Sauerstoffatom, an das $R_1^s$ gebunden ist, getrennt sind; ein 2-Methoxyethylrest; ein Phenyl- oder Benzylrest; ein Carboxyalkylrest mit insgesamt 2 bis 5 Kohlenstoffatomen; ein Alkoxycarbonylalkylrest mit 1 bis 4 Kohlenstoffatomen in der Alkoxygruppe und insgesamt 2 bis 5 Kohlenstoffatomen in der Carbonylalkylgruppe; oder ein 4-Pivaloylbenzylrest ist;

entweder:

$R_2^s$ ein Alkylrest mit 1 bis 4 Kohlenstoffatomen oder ein gegebenenfalls in Position 4 mit einem Pivaloylrest monosubstituierter Phenylrest ist;

$R_3^s$ ein 4-Pivaloylbenzoylrest ist;

oder:

$R_2^s$ ein 4-Pivaloylphenylrest ist und

$R_3^s$ ein Alkylcarbonylrest mit insgesamt 2 bis 5 Kohlenstoffatomen oder ein Benzoylrest ist.

**3.** Verbindung nach Anspruch 1 der Formel Ip

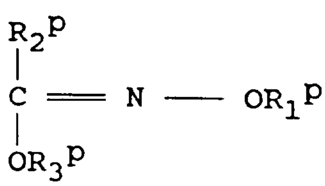

Ip

worin

$R_1^p$ ein Alkylrest mit 1 bis 4 Kohlenstoffatomen; ein Alkenylrest mit 3 bis 5 Kohlenstoffatomen, worin die Doppelbindung von dem Sauerstoffatom durch mindestens 2 Kohlenstoffatome getrennt ist; ein Mono-, Di- oder Trihydroxyalkylrest mit 2 bis 6

Kohlenstoffatomen, worin die Hydroxygruppen durch mindestens 2 Kohlenstoffatome von dem Sauerstoffatom, an das $R_1$ gebunden ist, getrennt sind; ein Mono-, Di- oder Trialkoxyrest mit 1 bis 4 Kohlenstoffatomen in der Alkoxygruppe und 2 bis 6 Kohlenstoffatomen in der Alkylengruppe, worin die Alkoxygruppen durch mindestens 2 Kohlenstoffatome von dem Sauerstoffatom, an das $R_1$ gebunden ist, getrennt sind; oder ein Phenyl- oder geradkettiger Phenylalkylrest mit 7 bis 11 Kohlenstoffatomen, der gegebenenfalls jeweils am Phenylring mono- oder unabhängig disubstituiert sein kann mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen, ist; und

$R_2{}^p$ und $R_3{}^p$ die oben für $R_2$ bzw. $R_3$ angegebene Bedeutung haben.

4. Verbindung nach Anspruch 1, die 4-Pivaloylbenzoyl-N-methoxy-4-pivaloylbenzimidat ist.

5. Verbindung nach Anspruch 1, die 4-Pivaloylbenzoyl-N-(2-hydroxyethoxy)-4-pivaloylbenzimidat oder 4-Pivaloylbenzoyl-N-carboxymethoxy-4-pivaloylbenzimidat oder eine Verbindung der Formel I ist, worin $R_1$, $R_2$ bzw. $R_3$ entweder 2,3-Di-OH-propyl-, 4-Pivaloylphenyl- bzw. 4-Pivaloylbenzoylreste, oder 2-OH-Ethyl-, 4-Pivaloylphenyl- bzw. 4-Pivaloylbenzoylreste, oder Isopropyl-, 4-Pivaloylphenyl- bzw. 4-Pivaloylbenzoylreste, oder Phenyl-, 4-Pivaloylphenyl- bzw. 4-Pivaloylbenzoylreste, oder Benzyl-, 4-Pivaloylphenyl- bzw. 4-Pivaloylbenzoylreste, oder Allyl-, 4-Pivaloylphenyl- bzw. 4-Pivaloylbenzoylreste, oder Methyl-, Phenyl- bzw. 4-Pivaloylbenzoylreste, oder Methyl-, t-Butyl- bzw. 4-Pivaloylbenzoylreste, oder Methyl-, Methyl- bzw. 4-Pivaloylbenzoylreste, oder Methyl-, 4-Pivaloylphenyl- bzw. Pivaloylreste, oder Methyl-, 4-Pivaloylphenyl- bzw. Benzoylreste, oder Methyl-, 4-Pivaloylphenyl- bzw. Acetylreste, oder Ethyl-, 4-Pivaloylphenyl- bzw. 4-Pivaloylbenzoylreste, oder t-Butyl-, 4-Pivaloylphenyl- bzw. 4-Pivaloylbenzoylreste, oder 2-MeO-Ethyl-, 4-Pivaloylphenyl- bzw. 4-Pivaloylbenzoylreste, oder Methyl-, 4-OH-Phenyl- bzw. 4-Pivaloylbenzoylreste, oder Methyl-, 4-Cl-Phenyl- bzw. 4-Pivaloylbenzoylreste, oder Methyl-, 4-Me-Phenyl- bzw. 4-Pivaloylbenzoylreste, oder Methyl-, 4MeO-Phenyl- bzw. 4-Pivaloylbenzoylreste, oder Methyl-, 4-Pivaloylphenyl- bzw. Benzylreste, oder t-Butoxycarbonylmethyl-, 4-Pivaloylphenyl- bzw. 4-Pivaloylbenzoylreste, oder t-Butoxycarbonylpropyl-, 4-Pivaloylphenyl- bzw. 4-Pivaloylbenzoylreste, oder t-Butoxycarbonylprop-2-yl-, 4-Pivaloylphenyl- bzw. 4-Pivaloylbenzoylreste, oder Carboxypropyl-, 4-Pivaloylphenyl- bzw. 4-Pivaloylbenzoylreste, oder 2-Carboxy-2-propyl-, 4-Pivaloylphenyl- bzw. 4-Pivaloylbenzoylreste, oder 4-Pivaloylbenzyl, 4-Pivaloylphenyl- bzw. 4-Pivaloylbenzoylreste sind, je nachdem in freier Form oder in Salzform oder in Form eines pharmazeutisch annehmbaren und physiologisch hydrolysierbaren Esters.

6. Verfahren zur Herstellung einer Verbindung nach Anspruch 1, umfassend, daß man
   a) eine Verbindung der Formel II

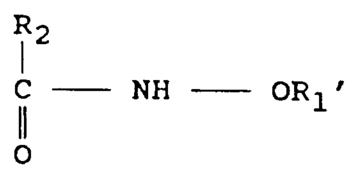

II

worin
$R_1'$ die in Anspruch 1 für $R_1$ angegebene Bedeutung hat, wobei jedoch die Hydroxygruppen irgendeines Hydroxyalkyl- oder Hydroxyphenylsubstituenten gegebenenfalls in geschützter Form sind, und
$R_2$ wie in Anspruch 1 definiert ist,
mit einer Verbindung der Formel III

$R_3 - X$ III

worin
$R_3$ wie in Anspruch 1 definiert ist und
X eine reaktive Gruppe ist,
umsetzt, oder

b) zur Herstellung einer Verbindung der Formel Ia

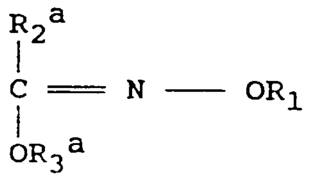

Ia

worin

$R_1$ wie in, Anspruch 1 definiert ist;

$R_2{}^a$ ein 4-Pivaloylphenylrest ist und

$R_3{}^a$ ein 4-Pivaloylbenzoylrest ist,

eine Verbindung der Formel IVa

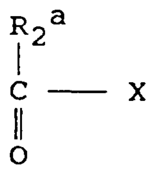

IVa

worin $R_2{}^a$ und X wie in diesem Anspruch definiert sind, mit einem tertiären Amin der Formel V

$R_4R_5R_6N$ V

worin $R_4$, $R_5$ und $R_6$ unabhängig Niedrigalkyl- oder Arylreste sind, und einer Verbindung der Formel VI

$H_2N — OR_1'$ VI

worin $R_1'$ wie in diesem Anspruch definiert ist, umsetzt und, falls notwendig, bei irgendwelchen geschützten Hydroxyalkyl- oder Hydroxyphenylsubstituenten die Schutzgruppe abspaltet und die entstehende Verbindung der Formel I je nachdem in freier Form oder Salzform oder in Form des pharmazeutisch annehmbaren und physiologisch hydrolysierbaren Esters gewinnt.

**7.** Pharmazeutische Zusammensetzung, umfassend eine Verbindung nach Anspruch 1 in freier Form oder in Form eines pharmazeutisch annehmbaren Salzes oder in Form eines pharmazeutisch und physiologisch hydrolysierbaren Esters zusammen mit einem pharmazeutisch annehmbaren Träger oder Verdünnungsmittel.

**8.** Verbindung nach Anspruch 7 zur Verwendung als Antidiabetikum und Mittel zur Absenkung des Blutcholesterin- und -triglyceridgehaltes.

**9.** Verwendung einer Verbindung nach Anspruch 7 zur Herstellung eines Arzneimittels zur Behandlung von Diabetes und zur Absenkung des Blutcholesterin- und -triglyceridgehaltes.

**Patentansprüche für folgende Vertragsstaaten : ES, GR**

**1.** Verfahren zur Herstellung einer Verbindung der Formel I

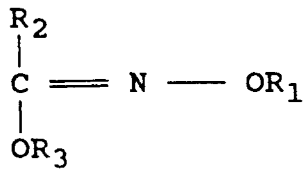

I

worin

$R_1$ ein Alkylrest mit 1 bis 6 Kohlenstoffatomen; ein Alkenylrest mit 3 bis 5 Kohlenstoffatomen, worin die Doppelbindung von dem Sauerstoffatom durch mindestens 2 Kohlenstoffatome getrennt ist; ein Mono- oder Polyhydroxyalkylrest mit 2 bis 8 Kohlenstoffatomen mit bis zu 7 Hydroxygruppen, worin die Hydroxygruppen durch mindestens 2 Kohlenstoffatome von dem Sauerstoff, an das $R_1$ gebunden ist, getrennt sind; ein Mono- oder Polyalkoxyalkylrest mit 1 bis 4 Kohlenstoffatomen in den Alkoxygruppen und 2 bis 8 Kohlenstoffatomen in der Alkylengruppe mit bis zu 7 Alkoxygruppen, worin die Alkoxygruppen durch mindestens 2 Kohlenstoffatome von dem Sauerstoff, an das $R_1$ gebunden ist, getrennt sind; ein Phenyl- oder geradkettiger Phenylalkylrest mit 7 bis 11 Kohlenstoffatomen, der gegebenenfalls jeweils am Phenylring mono- oder unabhängig disubstitutiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen; ein Carboxyalkylrest mit insgesamt 2 bis 6 Kohlenstoffatomen; ein Alkoxycarbonylalkylrest mit 1 bis 4 Kohlenstoffatomen in der Alkoxygruppe und insgesamt 2 bis 6 Kohlenstoffatomen in der Carbonylalkylgruppe; oder ein 4-Pivaloylbenzylrest ist;

entweder:

$R_2$ ein Alkylrest mit 1 bis 4 Kohlenstoffatomen; ein Phenyl- oder geradkettiger Phenylalkylrest mit 7 bis 11 Kohlenstoffatomen, der gegebenenfalls jeweils am Phenylring mono- oder unabhängig disubstituiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen oder in Position 4 des Phenylrings mit einem Pivaloylrest monosubstituiert ist, ist; und

$R_3$ ein 4-Pivaloylbenzoylrest ist;

oder

$R_2$ ein 4-Pivaloylphenylrest ist; und

$R_3$ ein Alkylrest mit 1 bis 4 Kohlenstoffatomen; ein geradkettiger Phenylalkylrest mit 7 bis 11 Kohlenstoffatomen, der am Phenylring gegebenenfalls entweder mono- oder unabhängig disubstituiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen oder in Position 4 des Phenylrings mit einem Pivaloylrest monosubstituiert ist; ein Alkylcarbonylrest mit insgesamt 2 bis 5 Kohlenstoffatomen; oder ein Benzoylrest, der gegebenenfalls mono- oder unabhängig disubstituiert ist mit Halogen mit einer Atomzahl von 9 bis 53, Hydroxyresten, Alkylresten mit 1 bis 4 Kohlenstoffatomen oder Alkoxyresten mit 1 bis 4 Kohlenstoffatomen, ist;

je nachdem in freier Form oder Salzform oder in Form eines pharmazeutisch annehmbaren und physiologisch hydrolysierbaren Esters,

umfassend, daß man

a) eine Verbindung der Formel II

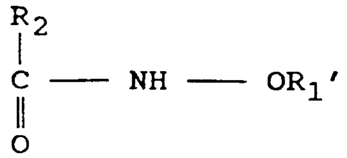

II

worin

$R_1'$ die in diesem Anspruch für $R_1$ angegebene Bedeutung hat, wobei jedoch die Hydroxygruppen irgendeines Hydroxyalkyl- oder Hydroxyphenylsubstituenten gegebenenfalls in geschützter Form sind, und

$R_2$ wie in diesem Anspruch definiert ist,

mit einer Verbindung der Formel III

$R_3 - X$ III

worin

$R_3$ wie in diesem Anspruch definiert ist und

X eine reaktive Gruppe ist,

umsetzt, oder

b) zur Herstellung einer Verbindung der Formel Ia

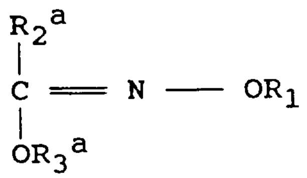

Ia

worin

$R_1$ wie in diesem Anspruch definiert ist;

$R_2{}^a$ ein 4-Pivaloylphenylrest ist und

$R_3{}^a$ ein 4-Pivaloylbenzoylrest ist,

eine Verbindung der Formel IVa

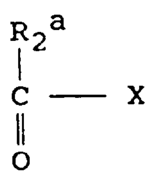

IVa

worin $R_2{}^a$ und X wie in diesem Anspruch definiert sind,

mit einem tertiären Amin der Formel V

$R_4R_5R_6N$ V

worin $R_4$, $R_5$ und $R_6$ unabhängig Niedrigalkyl- oder Arylreste sind,

und einer Verbindung der Formel VI

$H_2N - OR_1'$ VI

worin $R_1'$ wie in diesem Anspruch definiert ist,

umsetzt und, falls notwendig, bei irgendwelchen geschützten Hydroxyalkyl- oder Hydroxyphenylsubstituenten die Schutzgruppe abspaltet und die entstehende Verbindung der Formel I je nachdem in freier Form oder Salzform oder in Form des pharmazeutisch annehmbaren und physiologisch hydrolysierbaren Esters gewinnt.

**2.** Verfahren nach Anspruch 1 zur Herstellung der Verbindung der Formel I, die 4-Pivaloylbenzoyl-N-methoxy-4-pivaloylbenzimidat ist, umfassend, daß man

a) die Verbindung der Formel II, worin $R_1'$ ein Methylrest ist und $R_2$ ein 4-Pivaloylphenylrest ist, mit einer Verbindung der Formel III umsetzt, worin $R_3$ ein 4-Pivaloylbenzoylrest ist und X wie in Anspruch 1 definiert ist, oder

b) eine Verbindung der Formel IVa, worin $R_2{}^a$ ein 4-Pivaloylphenylrest ist und X wie in Anspruch 1 definiert ist, mit einem tertiären Amin der Formel V, worin $R_4$, $R_5$ und $R_6$ wie in Anspruch 1 definiert sind, und einer Verbindung der Formel VI, worin $R_1'$ ein Methylrest ist, umsetzt.

3. Verfahren nach Anspruch 2, umfassend, daß man
   a) N-Methoxy-4-pivaloylbenzamid mit 4-Pivaloylbenzoylchlorid umsetzt oder
   b) 4-Pivaloylbenzoylchlorid mit Triethylamin und Methoxyamin umsetzt.

4. Verfahren nach Anspruch 1, umfassend die Verwendung einer sterisch gehinderten organischen Base.

5. Verfahren nach Anspruch 4, umfassend die Verwendung von Triethylamin.

6. Verfahren zur Herstellung einer pharmazeutischen Zusammensetzung, umfassend, daR man eine Verbindung der Formel I, wie in Anspruch 1 definiert, in freier Form oder in Form des pharmazeutisch annehmbaren Salzes oder in Form des pharmazeutisch annehmbaren und physiologisch hydrolysierbaren Esters mit einem pharmazeutisch annehmbaren Träger oder Verdünnungsmittel vermischt.

**Revendications**

**Revendications pour les Etats contractants suivants : DE, GB, FR, IT, NL, SE, LI, CH, BE, AT, LU, DK**

1. Un composé de formule I

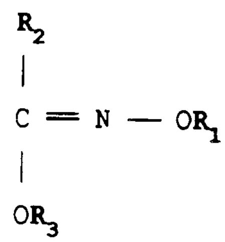

I

dans laquelle

$R_1$ signifie un groupe alkyle contenant de 1 à 6 atomes de carbone; un groupe alcényle contenant de 3 à 5 atomes de carbone et dans lequel la double liaison est séparée de l'atome d'oxygène par au moins 2 atomes de carbone; un groupe mono- ou polyhydroxyalkyle contenant de 2 à 8 atomes de carbone et ayant jusqu'à 7 groupes hydroxy, les groupes hydroxy étant séparés de l'atome d'oxygène auquel $R_1$ est lié par au moins 2 atomes de carbone ; un groupe mono- ou polyalcoxyalkyle dans lequel les groupes alcoxy contiennent de 1 à 4 atomes de carbone et le groupe alkylène contient de 2 à 8 atomes de carbone et ayant jusqu'à 7 groupes alcoxy, les groupes alcoxy étant séparés de l'atome d'oxygène auquel $R_1$ est lié par au moins 2 atomes de carbone ; un groupe phényle ou phénylalkyle à chaîne droite contenant de 7 à 11 atomes de carbone, les deux groupes étant éventuellement mono- ou indépendamment disubstitués dans le cycle phényle par des halogènes ayant un nombre atomique de 9 à 53 et des groupes hydroxy, alkyle contenant de 1 à 4 atomes de carbone ou alcoxy contenant de 1 à 4 atomes de carbone; un groupe carboxyalkyle contenant au total de 2 à 6 atomes de carbone; un groupe alcoxycarbonylalkyle dans lequel le groupe alcoxy contient de 1 à 4 atomes de carbone et le groupe carbonylalkyle au total de 2 à 6 atomes de carbone; ou un groupe 4-pivaloylbenzyle,

$R_2$ signifie un groupe alkyle contenant de 1 à 4 atomes de carbone; ou un groupe phényle ou phénylalkyle à chaîne droite contenant de 7 à 11 atomes de carbone, les deux groupes étant éventuellement mono- ou indépendamment disubstitués dans le cycle phényle par des halogènes ayant un nombre atomique de 9 à 53 et des groupes hydroxy, alkyle contenant de 1 à 4 atomes de carbone ou alcoxy contenant de 1 à 4 atomes de carbone, ou monosubstitués en position 4 du cycle phényle par un groupe pivaloyle, et

$R_3$ signifie un groupe 4-pivaloylbenzoyle, ou bien

$R_2$ signifie un groupe 4-pivaloylphényle et

$R_3$ signifie un groupe alkyle contenant de 1 à 4 atomes de carbone; un groupe phénylalkyle à chaîne droite contenant de 7 à 11 atomes de carbone, éventuellement mono- ou indépendamment disubstitué dans le cycle phényle par des halogènes ayant un nombre atomique de 9 à 53 et des groupes hydroxy, alkyle contenant de 1 à 4 atomes de carbone ou alcoxy

contenant de 1 à 4 atomes de carbone, ou monosubstitué en position 4 du cycle phényle par un groupe pivaloyle; un groupe alkylcarbonyle contenant au total de 2 à 5 atomes de carbone; ou un groupe benzoyle éventuellement mono- ou indépendamment disubstitué par des halogènes ayant un nombre atomique de 9 à 53 et des groupes hydroxy, alkyle contenant de 1 à 4 atomes de carbone ou alcoxy contenant de 1 à 4 atomes de carbone,

sous forme libre ou sous forme d'un sel ou sous forme d'un ester pharmaceutiquement acceptable et physiologiquement hydrolysable lorsque cela est approprié.

**2.** Un composé selon là revendication 1 de formule Is

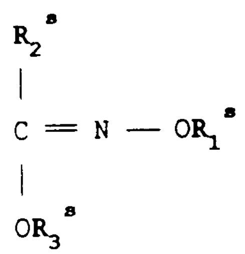

Is

dans laquelle

$R_1^s$ signifie un groupe alkyle contenant de 1 à 4 atomes de carbone; un groupe allyle; un groupe mono- ou dihydroxyalkyle contenant de 2 à 4 atomes de carbone, les groupes hydroxy étant séparés de l'atome d'oxygène auquel $R_1^s$ est lié par au moins 2 atomes de carbone; un groupe 2-méthoxyéthyle; un groupe phényle ou benzyle; un groupe carboxyalkyle contenant au total de 2 à 5 atomes de carbone; un groupe alcoxycarbonyl- alkyle dans lequel le groupe alcoxy contient de 1 à 4 atomes de carbone et le groupe carbonylalkyle au total de 2 à 5 atomes de carbone; ou un groupe 4-pivaloylbenzyle,

$R_2^s$ signifie un groupe alkyle contenant de 1 à 4 atomes de carbone ou un groupe phényle éventuellement mono-substitué en position 4 par un groupe pivaloyle,

$R_3^s$ signifie un groupe 4-pivaloylbenzoyle, ou bien

$R_2^s$ signifie un groupe 4-pivaloylphényle et

$R_3^s$ signifie un groupe alkylcarbonyle contenant au total de 2 à 5 atomes de carbone; ou un groupe benzoyle.

**3.** Un composé selon la revendication 1 de formule Ip

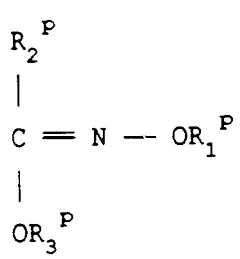

Ip

dans laquelle

$R_1^p$ signifie un groupe alkyle contenant de 1 à 4 atomes de carbone; un groupe alcényle contenant de 3 à 5 atomes de carbone dans lequel la double liaison est séparée de l'atome d'oxygène par au moins 2 atomes de carbone; un groupe mono-, di- ou trihydroxyalkyle contenant de 2 à 6 atomes de carbone, les groupes hydroxy étant séparés de l'atome d'oxygène auquel $R_1$ est lié par au moins 2 atomes de carbone; un groupe mono-, di- ou trialcoxyalkyle dans lequel les groupes alcoxy contiennent de 1 à 4 atomes de carbone et le groupe alkylène contient de 2 à 6 atomes de carbone, les groupes alcoxy étant séparés de l'atome d'oxygène auquel $R_1$ est lié par au moins 2 atomes de carbone; ou un groupe phényle ou phényl- alkyle à chaîne

droite contenant de 7 à 11 atomes de carbone, les deux groupes étant éventuellement mono- ou indépendamment disubstitués dans le cycle phényle par des halogènes ayant un nombre atomique de 9 à 53 et des groupes hydroxy, alkyle contenant de 1 à 4 atomes de carbone ou alcoxy contenant de 1 à 4 atomes de carbone, et

$R_2^p$ et $R_3^p$ ont la signification indiquée plus haut respectivement pour $R_2$ et $R_3$.

**4.** Le composé selon la revendication 1 qui est le 4-pivaloylbenzoyl-N-méthoxy-4-pivaloylbenzimidate.

**5.** Le composé selon la revendication 1 qui est

- le 4-pivaloylbenzoyl-N-(2-hydroxyéthoxy)-4-pivaloylbenzimidate ou
- le 4-pivaloylbenzoyl-N-carboxyméthoxy-4-pivaloylbenzimidate, ou bien
- qui répond à la formule I où $R_1$, $R_2$ et $R_3$ signifient respectivement un groupe
  - 2,3-di-OH-propyle, 4-pivaloylphényle et 4-pivaloylbenzoyle,
  - 2-OH-éthyle, 4-pivaloylphényle et 4-pivaloylbenzoyle,
  - isopropyle, 4-pivaloylphényle et 4-pivaloylbenzoyle,
  - phényle, 4-pivaloylphényle et 4-pivaloylbenzoyle,
  - benzyle, 4-pivaloylphényle et 4-pivaloylbenzoyle,
  - allyle, 4-pivaloylphényle et 4-pivaloylbenzoyle,
  - méthyle, phényle et 4-pivaloylbenzoyle,
  - méthyle, tert.-butyle et 4-pivaloylbenzoyle,
  - méthyle, méthyle et 4-pivaloylbenzoyle,
  - méthyle, 4-pivaloylphényle et pivaloyle,
  - méthyle, 4-pivaloylphényle et benzoyle,
  - méthyle, 4-pivaloylphényle et acétyle,
  - éthyle, 4-pivaloylphényle et 4-pivaloylbenzoyle,
  - tert.-butyle, 4-pivaloylphényle et 4-pivaloylbenzoyle,
  - 2-MeO-éthyle, 4-pivaloylphényle et 4-pivaloylbenzoyle,
  - méthyle, 4-OH-phényle et 4-pivaloylbenzoyle,
  - méthyle, 4-Cl-phényle et 4-pivaloylbenzoyle,
  - méthyle, 4-Me-phényle et 4-pivaloylbenzoyle,
  - méthyle, 4-MeO-phényle et 4-pivaloylbenzoyle,
  - méthyle, 4-pivaloylphényle et benzyle,
  - tert.-butoxycarbonylméthyle, 4-pivaloylphényle et 4-pivaloyl-benzoyle,
  - tert.-butoxycarbonylpropyle, 4-pivaloylphényle et 4-pivaloyl-benzoyle,
  - tert.-butoxycarbonylprop-2-yle, 4-pivaloylphényle et 4-pivaloyl-benzoyle,
  - carboxypropyle, 4-pivaloylphényle et 4-pivaloylbenzoyle,
  - 2-carboxy-2-propyle, 4-pivaloylphényle et 4-pivaloylbenzoyle, ou
  - 4-pivaloylbenzyle, 4-pivaloylphényle et 4-pivaloylbenzoyle,

sous forme libre ou sous forme d'un sel ou sous forme d'un ester pharmaceutiquement acceptable et physiologiquement acceptable lorsque cela est approprié.

**6.** Un procédé de préparation d'un composé selon la revendication 1, comprenant

a) la réaction d'un composé de formule II

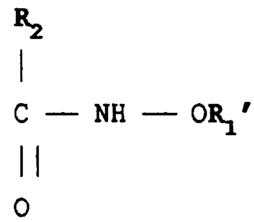

II

où

$R_1'$ a la signification indiquée à la revendication 1 pour $R_1$, mais avec les groupes hydroxy de n'importe quel substituant hydroxy-alkyle ou hydroxy-phényle éventuellement sous forme protégée, et

$R_2$ est tel que défini à la revendication 1,
avec un composé de formule III

$R_3 - X$ III

où
$R_3$ est tel que défini à la revendication 1, et
X signifie un groupe réactif, ou bien
b) pour la préparation d'un composé de formule Ia

$$\begin{array}{l} R_2^{a} \\ | \\ C = N - OR_1 \\ | \\ OR_3^{a} \end{array} \qquad \text{Ia}$$

où
$R_1$ est tel que défini a la revendication 1,
$R_2^a$ signifie un groupe 4-pivaloylphényle, et
$R_3^a$ signifie un groupe 4-pivaloylbenzoyle,
la réaction d'un composé de formule IVa

$$\begin{array}{l} R_2^{a} \\ | \\ C - X \\ || \\ O \end{array} \qquad \text{IVa}$$

où
$R_2^a$ et X sont tels que définis dans cette revendication,
avec une amine tertiaire de formule V

$R_4R_5R_6N$ V

où $R_4$, $R_5$, $R_6$ signifient indépendamment un groupe alkyle inférieur ou aryle, et un composé de formule VI

$H_2N - OR_1'$ VI

où $R_1'$ est tel que défini dans cette revendication,
et si nécessaire, la déprotection de n'importe quel substituant hydroxy-alkyle ou hydroxy-phényle protégé, et la récupération du composé résultant de formule I sous forme libre ou sous forme d'un sel ou d'un ester pharmaceutiquement acceptable et physiologiquement hydrolysable lorsque cela est approprié.

**7.** Une composition pharmaceutique comprenant un composé selon la revendication 1 sous forme libre ou sous forme d'un sel pharmaceutiquement acceptable ou d'un ester pharmaceutiquement acceptable et physiologiquement hydrolysable lorsque cela est approprié, en association avec un véhicule ou diluant pharmaceutiquement acceptable.

**8.** Un composé tel que défini à la revendication 7 pour l'utilisation comme agent anti-diabétique et agent abaissant les taux de cholestérol et de triglycérides dans le sang.

**9.** Utilisation d'un composé selon la revendication 7 pour la préparation d'un médicament pour le traitement du diabète et pour abaisser les taux de cholestérol et de triglycérides dans le sang.

**Revendications pour les Etats contractants suivants : ES, GR**

**1.** Un procédé de préparation d'un composé de formule I

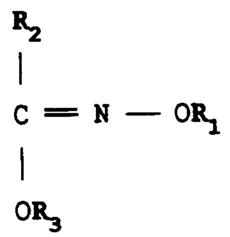

I

dans laquelle

$R_1$ signifie un groupe alkyle contenant de 1 à 6 atomes de carbone; un groupe alcényle contenant de 3 à 5 atomes de carbone dans lequel la double liaison est séparée de l'atome d'oxygène par au moins 2 atomes de carbone; un groupe mono- ou polyhydroxyalkyle contenant de 2 à 8 atomes de carbone et ayant jusqu'à 7 groupes hydroxy, les groupes hydroxy étant séparés de l'atome d'oxygène auquel $R_1$ est lié par au moins 2 atomes de carbone; un groupe mono- ou polyalcoxyalkyle dans lequel les groupes alcoxy contiennent de 1 à 4 atomes de carbone et le groupe alkylène contient de 2 à 6 atomes de carbone et ayant jusqu'à 7 groupes alcoxy, les groupes alcoxy étant séparés de l'atome d'oxygène auquel $R_1$ est lié par au moins 2 atomes de carbone ; un groupe phényle ou phénylalkyle à chaîne droite contenant de 7 à 11 atomes de carbone, les deux groupes étant éventuellement mono- ou indépendamment disubstitués dans le cycle phényle par des halogènes ayant un nombre atomique de 9 à 53 et des groupes hydroxy, alkyle contenant de 1 à 4 atomes de carbone ou alcoxy contenant de 1 à 4 atomes de carbone; un groupe carboxyalkyle contenant au total de 2 à 6 atomes de carbone; un groupe alcoxycarbonylalkyle dans lequel le groupe alcoxy contient de 1 à 4 atomes de carbone et le groupe carbonylalkyle au total de 2 à 6 atomes de carbone; ou un groupe 4-pivaloylbenzyle,

$R_2$ signifie un groupe alkyle contenant de 1 à 4 atomes de carbone; ou un groupe phényle ou phénylalkyle à chaîne droite contenant de 7 à 11 atomes de carbone, les deux groupes étant éventuellement mono- ou indépendamment disubstitués dans le cycle phényle par des halogènes ayant un nombre atomique de 9 à 53 et des groupes hydroxy, alkyle contenant de 1 à 4 atomes de carbone ou alcoxy contenant de 1 à 4 atomes de carbone, ou monosubstitués en position 4 du cycle phényle par un groupe pivaloyle, et

$R_3$ signifie un groupe 4-pivaloylbenzoyle, ou bien

$R_2$ signifie un groupe 4-pivaloylphényle et

$R_3$ signifie un groupe alkyle contenant de 1 à 4 atomes de carbone; un groupe phénylalkyle à chaîne droite contenant de 7 à 11 atomes de carbone, éventuellement mono- ou indépendamment disubstitué dans le cycle phényle par des halogènes ayant un nombre atomique de 9 à 53 et des groupes hydroxy, alkyle contenant de 1 à 4 atomes de carbone ou alcoxy contenant de 1 à 4 atomes de carbone, ou monosubstitué en position 4 du cycle phényle par un groupe pivaloyle; un groupe alkylcarbonyle contenant au total de 2 à 5 atomes de carbone; ou un groupe benzoyle éventuellement mono- ou indépendamment disubstitué par des halogènes ayant un nombre atomique de 9 à 53 et des groupes hydroxy, alkyle contenant de 1 à 4 atomes de carbone ou alcoxy contenant de 1 à 4 atomes de carbone,

sous forme libre ou sous forme d'un sel ou sous forme d'un ester pharmaceutiquement acceptable et physiologiquement hydrolysable lorsque cela est approprié, lequel procédé comprend

a) la réaction d'un composé de formule II

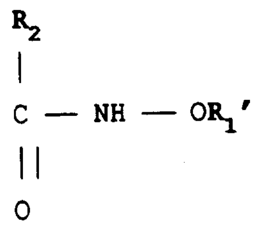

II

où

$R_1'$ a la signification indiquée dans cette revendication pour $R_1$, mais avec les groupes hydroxy de n'importe quel substituant hydroxy-alkyle ou hydroxyphényle éventuellement sous forme protégée, et

$R_2$ est tel que défini dans cette revendication,

avec un composé de formule III

$R_3 - X$ III

où

$R_3$ est tel que défini dans cette revendication, et

X signifie un groupe réactif, ou bien

b) pour la préparation d'un composé de formule Ia

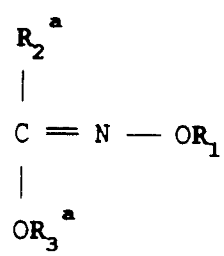

Ia

où

$R_1$ est tel que défini dans cette revendication,

$R_2{}^a$ signifie un groupe 4-pivaloylphényle, et

$R_3{}^a$ signifie un groupe 4-pivaloylbenzoyle,

la réaction d'un composé de formule IVa

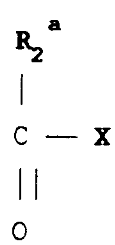

IVa

où

$R_2{}^a$ et X sont tels que définis dans cette revendication,

avec une amine tertiaire de formule V

$R_4R_5R_6N$ V

où $R_4$, $R_5$, $R_6$ signifient indépendamment un groupe alkyle inférieur ou aryle, et un composé de formule VI

$H_2N — OR_1'$ VI

où $R_1'$ est tel que défini dans cette revendication,
et, si nécessaire, la déprotection de n'importe quel substituant hydroxy-alkyle ou hydroxy-phényle protégé, et la récupération du composé résultant de formule I sous forme libre ou sous forme d'un sel ou d'un ester pharmaceutiquement acceptable et physiologiquement hydrolysable lorsque cela est approprié.

2. Un procédé selon la revendication 1 pour la préparation d'un composé de formule I qui est le 4-pivaloylbenzoyl-N-méthoxy-4-pivaloylbenzimidate, comprenant
   a) la réaction du composé de formule II où $R_1'$ signifie un groupe méthyle et $R_2$ signifie un groupe 4-pivaloylphényle, avec un composé de formule III où $R_3$ signifie un groupe 4-pivaloylbenzoyle et X est tel que défini à la revendication 1, ou bien
   b) la réaction d'un composé de formule IVa où $R_2^a$ signifie un groupe 4-pivaloylphényle et X est tel que défini à la revendication 1, avec une amine tertiaire de formule V où $R_4$, $R_5$ et $R_6$ sont tels que définis à la revendication 1, et un composé de formule VI où $R_1'$ signifie un groupe méthyle.

3. Un procédé selon la revendication 2, comprenant
   a) la réaction du N-méthoxy-4-pivaloylbenzamide avec le chlorure de 4-pivaloylbenzoyle, ou bien
   b) la réaction du chlorure de 4-pivaloylbenzoyle avec la triéthylamine et la méthoxyamine.

4. Un procédé selon la revendication 1, comprenant l'utilisation d'une base organique encombrée.

5. Un procédé selon la revendication 4, comprenant l'utilisation de la triéthylamine.

6. Un procédé de préparation d'une composition pharmaceutique, comprenant le mélange d'un composé de formule I tel que défini à la revendication 1 sous forme libre ou sous forme d'un sel pharmaceutiquement acceptable ou d'un ester pharmaceutiquement acceptable et physiologiquement hydrolysable lorsque cela est approprié, en association avec un véhicule ou diluant pharmaceutiquement acceptable.